# EUROPEAN PATENT SPECIFICATION

(11) **EP 4 154 854 B1**
(45) Date of publication and mention of the grant of the patent: **27.11.2024**
(21) Application number: 21834071.9
(22) Date of filing: 22.06.2021
(51) Int. Cl.: A61F 13/53, A61F 13/532, A61F 13/533, A61F 13/537, A61F 13/535, A61F 13/536

(54) **ABSORBENT ARTICLE**
ABSORBIERENDER ARTIKEL
ARTICLE ABSORBANT

(30) Priority: 30.06.2020 JP 2020113599
(43) Date of publication of application: 29.03.2023
(73) Proprietor: Unicharm Corporation, Shikokuchuo-shi, Ehime 799-0111 (JP)
(72) Inventor: ONISHI, Kazuaki, Kanonji-shi, Kagawa 769-1602 (JP)
(74) Representative: Staeger & Sperling Partnerschaftsgesellschaft mbB
(86) International application number: PCT/JP2021/023659
(87) International publication number: WO 2022/004499

(56) References cited:
- EP-A1- 3 738 563
- JP-A- 2012 143 544
- JP-A- 2012 143 544
- JP-A- 2017 012 437
- JP-A- 2017 012 437
- JP-A- 2018 000 525
- JP-A- 2018 000 525
- JP-A- 2018 000 872
- JP-A- 2019 118 722

## Description

### FIELD

The present invention relates to an absorbent article.

### BACKGROUND

There are known absorbent articles that exhibit excellent absorption performance (for example, absorption rate, and liquid return suppression) even when the absorbent articles repeatedly absorb bodily fluids. For example, Patent Literature 1 discloses an absorbent article including an absorbent core including superabsorbent polymer particles. In this absorbent article, the absorbent core is formed of a non-skin side layer on a non-skin facing surface side and a skin side layer on a skin facing surface side. The non-skin side layer includes a plurality of groove portions including a plurality of main groove portions that extend in a lengthwise direction and penetrate the non-skin side layer in a thickness direction, and a plurality of bases that extend in the lengthwise direction, and each of the plurality of main groove portions and each of the plurality of bases alternately extend in a lateral direction. The skin side layer includes a plurality of main groove portion corresponding portions and a plurality of base corresponding portions arranged at positions overlapping the plurality of main groove portions and the plurality of bases in the thickness direction, respectively. Both an average density of the superabsorbent polymer particles contained in each of the plurality of base portions and an average density of the superabsorbent polymer particles contained in each of the plurality of main groove portion corresponding portions are lower than an average density of the superabsorbent polymer particles contained in each of the plurality of base corresponding portions.

### [CITATION LIST]

### [PATENT LITERATURE]

Patent Literature 1: Japanese Unexamined Patent Publication No. 2018-872

Further prior art in this technical field is disclosed in documents JP 2012 143544 A and JP 2019 118722 A.

### SUMMARY

### [TECHNICAL PROBLEM]

An absorbent article as described in Patent Literature 1 includes a main groove portion and a base adjacent to the main groove portion in the non-skin side layer on the non-skin facing surface side. Therefore, the bodily fluid (for example: urine) that has reached the non-skin side layer through the skin side layer can be diffused in the main groove portion in the lengthwise direction, can be allowed to penetrate into the base and diffuse in the lateral direction, and can be transferred from the base to the base corresponding portion to be held in the base corresponding portion. Due to that, the entire absorbent core can be effectively used for absorption of the bodily fluid. Further, since the superabsorbent polymer particles are contained in the base and swell due to absorption of the bodily fluid, the shape of the main groove portion in the thickness direction can be maintained.

In such an absorbent article, part of the bodily fluid that has passed through the main groove portion corresponding portion before reaching the main groove portion is absorbed by the main groove portion corresponding portion, and can be held to the base corresponding portion from the main groove portion corresponding portion. In addition, the bodily fluid that has reached the main groove portion is reduced by the amount absorbed by the main groove portion corresponding portion, the ratio of the bodily fluid absorbed from the main groove portion to the base to the bodily fluid that is diffused in the main groove portion in the lengthwise direction increases. The bodily fluid that has absorbed to the base can be transferred from the base to the base corresponding portion and held in the base corresponding portion. Therefore, in a conventional absorbent article, it can be said that the ratio of the bodily fluid held in the base corresponding portion is high compared with a case of diffusion of the bodily fluid. Therefore, in a case where a bodily fluid is repeatedly absorbed, the degree of swelling of the superabsorbent polymer particles in the base becomes excessively large, the local thickness is increased, and there is a risk that the feeling of wearing may deteriorate.

An aspect of the present invention is to provide an absorbent article capable of maintaining absorption performance and a feeling of wearing even when a bodily fluid is repeatedly absorbed.

### [SOLUTION TO PROBLEM]

The present invention provides an absorbent article having a lengthwise direction, a lateral direction, and a thickness direction, the absorbent article comprising: an absorbent core that contains superabsorbent polymer particles, wherein the absorbent core has a skin facing surface, and a non-skin facing surface, and is formed of a skin side layer on a skin facing surface side, and a non-skin side layer on a non-skin facing surface side, the skin side layer includes a plurality of groove portions that extend in the lengthwise direction and penetrate in the thickness direction, and a plurality of bases that extend in the lengthwise direction, each of the plurality of groove portions and each of the plurality of bases alternately extend in the lateral direction, the plurality of groove portions includes a plurality of main groove portions that extend in the lengthwise direction, and a plurality of sub-groove portions that are present in communication with each of the plurality of main groove portions through base ends with predetermined spaces in a direction crossing each of the plurality of main groove portions, the non-skin side layer includes a plurality of groove portion corresponding portions and a plurality of base corresponding portions at positions that overlap the plurality of groove portions and the plurality of bases in the thickness direction, respectively, and a first average density of the superabsorbent polymer particles contained in each of the plurality of groove portion corresponding portions and the plurality of base corresponding portions is lower than a second average density of the superabsorbent polymer particles contained in each of the plurality of bases.

### [ADVANTAGEOUS EFFECT OF THE INVENTION]

The absorbent article according to the present invention can maintain absorption performance and a feeling of wearing even when a bodily fluid is repeatedly absorbed.

### BRIEF DESCRIPTION OF DRAWINGS

FIG. 1 is a plan view of an absorbent article according to a first embodiment.
FIG. 2 is a plan view of an absorbent body of the absorbent article according to the first embodiment.
FIG. 3 is a cross-sectional view taken along a line III-III in FIG. 2 according to the first embodiment.
FIG. 4 is a plan view of an absorbent core of the absorbent article according to the first embodiment.
FIG. 5 is a cross-sectional view taken along a line V-V in FIG. 2 according to the first embodiment.
FIG. 6 is a schematic view showing the flow of a bodily fluid in a cross section taken along a line III-III in FIG. 2 according to the first embodiment.
FIG. 7 is a cross-sectional view taken along a line III-III in FIG. 2 according to a second embodiment.
FIG. 8 is a plan view of an absorbent core of an absorbent article according to a modified example of the second embodiment.
FIG. 9 is a schematic view showing a manufacturing apparatus used in a method for manufacturing the absorbent article according to the first and second embodiments.
FIG. 10 is a schematic view showing a state in which an absorbent material is supplied on a suction drum of a manufacturing apparatus.

### DESCRIPTION OF EMBODIMENTS

Embodiments of the present invention relate to the following aspects.

### [Aspect 1]

An absorbent article having a lengthwise direction, a lateral direction, and a thickness direction, the absorbent article comprising:
an absorbent core that contains superabsorbent polymer particles, wherein
the absorbent core has a skin facing surface, and a non-skin facing surface, and is formed of a skin side layer on a skin facing surface side, and a non-skin side layer on a non-skin facing surface side,
the skin side layer includes
   a plurality of groove portions that extend in the lengthwise direction and penetrate in the thickness direction, and
   a plurality of base portions that extend in the lengthwise direction,
each of the plurality of groove portions and each of the plurality of base portions alternately extend in the lateral direction,
the plurality of groove portions include
   a plurality of main groove portions that extend in the lengthwise direction, and
   a plurality of sub-groove portions that are present in communication with each of the plurality of main groove portions through base ends with predetermined spaces in a direction crossing each of the plurality of main groove portions,
the non-skin side layer includes a plurality of groove portion corresponding portions and a plurality of base corresponding portions at positions that overlap the plurality of groove portions and the plurality of base portions in the thickness direction, respectively, and
a first average density of the superabsorbent polymer particles contained in each of the plurality of groove portion corresponding portions and the plurality of base corresponding portions is lower than a second average density of the superabsorbent polymer particles contained in each of the plurality of base portions.

In the present absorbent article, an excreted bodily fluid is transferred in the lengthwise direction of the absorbent core through the main groove portions. The bodily fluid is absorbed into the inside of the absorbent core from the main groove portion and the sub-groove portion communicating with the main groove portion. Therefore, in the present absorbent article, even when the bodily fluid is repeatedly absorbed, the absorption performance, for example, absorption rate, can be maintained. The first average density of the superabsorbent polymer particles contained in the groove portion corresponding portion and the base corresponding portion is lower than the second average density of the superabsorbent polymer particles contained in the base potion. Therefore, while the bodily fluid absorbed into the groove portion corresponding portion is diffused in the lengthwise direction, part of the bodily fluid is transferred to the base corresponding portion. That is, since only part of the bodily fluid that has been transferred to the base corresponding portion in the bodily fluid absorbed into the groove portion corresponding portion is transferred to the base potion and can be held in the base potion, the superabsorbent polymer particles in the base potion can be prevented from swelling and becoming excessively large. As a result, in the present absorbent article, even when the bodily fluid is repeatedly absorbed, a local increase in the thickness is suppressed, and a feeling of wearing can be maintained.

### [Aspect 2]

The absorbent article according to aspect 1, wherein
a ratio of the superabsorbent polymer particles contained in the plurality of groove portion corresponding portions and the plurality of base corresponding portions to the superabsorbent polymer particles contained in the entire absorbent core is less than 30%.

In the present absorbent article, the ratio of the superabsorbent polymer particles contained in the plurality of groove portion corresponding portions and the plurality of base corresponding portions to the superabsorbent polymer particles contained in the entire absorbent core is less than 30%. Therefore, since the superabsorbent polymer particles contained in the plurality of groove portion corresponding portions and the plurality of base corresponding portions can be sufficiently reduced compared with the superabsorbent polymer particles contained the base portions, the swelling of the groove portion corresponding portion and the base corresponding portion is suppressed. Therefore, in the present absorbent article, an increase in the thickness can be more reliably suppressed. Further, in the present absorbent article, the bodily fluid can be more rapidly diffused in the lengthwise direction and the lateral direction through the groove portion corresponding portion and the base corresponding portion.

### [Aspect 3]

The absorbent article according to aspect 1 or 2, wherein
each of the plurality of base portions has a surface layer region including base side surfaces that are in contact with the plurality of groove portions, and a top surface that is positioned between the base side surfaces, and
an average density of the superabsorbent polymer particles contained in the surface layer region is lower than the second average density.

In the present absorbent article, the average density of the superabsorbent polymer particles contained in the surface layer region is lower than the second average density. Therefore, the bodily fluid in the groove portion infiltrates into the surface layer region in which the average density of the superabsorbent polymer particles is lower, and thereby more rapidly permeates into the base potion. Therefore, the present absorbent article can rapidly absorb the bodily fluid in the groove portion. Further, the surface layer region in which there are few superabsorbent polymer particles is less likely to cause a phenomenon that the superabsorbent polymer particles that have absorbed water and swollen spill into the groove portion and block the groove portion. Therefore, the present absorbent article can maintain a rapid absorption rate even when the absorbent article repeatedly absorbs the bodily fluid.

### [Aspect 4]

The absorbent article according to any one of aspects 1 to 3, wherein
in an excretion region of the absorbent core, a space between the plurality of main groove portions arranged on both sides in the lateral direction with a center of the absorbent core in the lateral direction interposed therebetween is 15% or more and less than 45% of a minimum length of the absorbent core in the lateral direction in the excretion region.

Since the space between the main groove portions is 15% or more and less than 45% of the minimum length of the absorbent core in the lateral direction in the excretion region, the present absorbent article can prevent the bodily fluid from concentrating in the center of the absorbent core, and can disperse the bodily fluid and more rapidly absorb the bodily fluid from the groove portions.

### [Aspect 5]

The absorbent article according to any one of aspects 1 to 4, wherein
the plurality of base portions, the plurality of groove portion corresponding portions, and the plurality of base corresponding portions are integrated.

Since the bodily fluid can move more smoothly between the groove portion corresponding portion, the base corresponding portion, and the base potion, the bodily fluid can be more rapidly diffused.

### [Aspect 6]

The absorbent article according to any one of aspects 1 to 5, wherein
an average density of the superabsorbent polymer particles contained in a central region including a center of each of the plurality of base portions in the lateral direction is higher than the second average density.

Since the average density of the superabsorbent polymer particles contained in the central region including the center of the base potion is higher than the second average density, the swelling of the base side surface in contact with the groove portion is suppressed, and the collapse of the groove portion can be suppressed. Therefore, in the present absorbent article, even in a case where the bodily fluid is repeatedly absorbed, the bodily fluid can more reliably permeate from the groove portion to the groove portion corresponding portion and be absorbed.

### [Aspect 7]

The absorbent article according to any one of aspects 1 to 6, wherein
the plurality of base portions include
a plurality of narrow portions in which the plurality of sub-groove portions that are present in communication with each of the plurality of main groove portions adjacent to each other in the lateral direction face each other and which are present between the plurality of sub-groove portions facing each other, and
a plurality of wide portions which are present in a section of the plurality of base portions excluding the plurality of narrow portions.

The bodily fluid permeates from the main groove portion to the groove portion corresponding portion, and also permeates into the narrow portion through the sub-groove portion communicating with the main groove portion. Since the bodily fluid that has permeated from the groove portion to the groove portion corresponding portion and the narrow portion is absorbed into the inside of the absorbent core, the absorption performance, for example, absorption rate, can be maintained in the present absorbent article even when the bodily fluid is repeatedly absorbed.

### [Aspect 8]

The absorbent article according to any one of aspects 1 to 7, wherein
the plurality of groove portion corresponding portions include
   a plurality of main groove portion corresponding portions corresponding to the plurality of main groove portions, and
   a plurality of sub-groove portion corresponding portions corresponding to the plurality of sub-groove portions, and
the first average density of the plurality of main groove portion corresponding portions is lower than the second average density.

The first average density of the main groove portion corresponding portion is lower than the second average density. While the bodily fluid that has absorbed into the main groove portion corresponding portion is diffused in the lengthwise direction through the main groove portion corresponding portion, the bodily fluid is transferred to the base corresponding portion. That is, only part of the bodily fluid that has transferred to the base corresponding portion in the bodily fluid that has absorbed into the main groove portion corresponding portion is transferred to the base potion, and can be held in the base potion. Therefore, in the present absorbent article, the superabsorbent polymer particles in the base potion can be prevented from swelling and becoming excessively large.

### [Aspect 9]

The absorbent article according to aspect 8, wherein
the first average density of the plurality of sub-groove portion corresponding portions is lower than the second average density.

Since the first average density of the sub-groove portion corresponding portion as well as the main groove corresponding portion is also lower than the second average density, the above-described effect can be more reliably obtained.

### [Aspect 10]

The absorbent article according to aspect 6, wherein
each of the plurality of base portions is continuous from one end to the other end of the absorbent core in the lengthwise direction.

In the present absorbent article, since the central region is continuous from one end to the other end of the absorbent core in the lengthwise direction, the swelling of the base side surface that is in contact with the groove portion is suppressed across the entire region of the absorbent core in the lengthwise direction, the collapse of the groove portion can be suppressed, and the bodily fluid can be continuously and repeatedly diffused in the lengthwise direction.

Hereinafter, an absorbent article according to an embodiment will be described with reference to drawings.

In the present specification, unless otherwise specified, "viewing an object (for example, an absorbent article, an absorbent body, or an absorbent core) placed on a horizontal plane in an unfolded state from above or below the object in the thickness direction" will be referred to as a "plan view". In a case where the object is an absorbent article, viewing the absorbent article in the thickness direction from a top-surface sheet side in an unfolded state may be simply referred to as a "plan view". In a case where the object is an absorbent core, viewing the absorbent article in the thickness direction from a non-skin facing surface side of the absorbent core in an unfolded state may be simply referred to as a "plan view".

Various directions and the like used in the present specification are as follows, unless otherwise specified. The "longitudinal direction" refers to "a direction in which the length of a longitudinally long object in a plan view is long", the "lateral direction" refers to "a direction in which the length of the longitudinally long object in a plan view is short", and the "thickness direction" refers to "a direction vertical to the object placed on a horizontal plane in an unfolded state". The longitudinal direction, the lateral direction, and the thickness direction are in a relationship that is orthogonal to each other. It should be noted that, although each direction includes two opposite directions, when shown in the drawings, there is a case where only one direction is shown with respect to a direction orthogonal to the paper surface.

In the present specification, unless otherwise specified, in the thickness direction of the absorbent article, a "proximal side relative to the skin surface of the wearer while the absorbent article is put on" is referred to as a "skin facing surface side", and a "distal side relative to the skin surface of the wearer while the absorbent article is put on" is referred to as a "non-skin facing surface side". In the present specification, the "skin facing surface side surface" and the "non-skin facing surface side surface" of various members (for example, a top-surface sheet, an absorbent body, and a back-surface sheet) that constitute the absorbent article are simply referred to as a "skin facing surface" and a "non-skin facing surface," respectively.

In the present specification, the "main groove portion" means not only a groove portion that extends in a direction parallel to the longitudinal direction, but also a groove portion that extends along the longitudinal direction. An angle formed between the main groove portion and the longitudinal direction is preferably less than 45°, more preferably less than 30°, and even more preferably less than 15°. The main groove portion can extend linearly, or non-linearly, for example, in a curved shape. In the present specification, the width of the main groove portion means the length of the main groove portion in a direction orthogonal to a direction in which the main groove portion extends in an object portion.

In the present specification, the "sub-groove portion" means not only a groove portion that extends in a direction parallel or substantially parallel to the lateral direction, but also a groove portion that extends in a direction other than the longitudinal direction. An angle formed between the sub-groove portion and the main groove portion (or the tangent line of the main groove portion) at a portion (base end) where the sub-groove portion communicates with the main groove portion is preferably 45° or more, more preferably 60° or more, and even more preferably 80° or more. The sub-groove portion can extend linearly, or non-linearly, for example, in a curved shape. In the present specification, the width of the sub-groove portion means the length of the sub-groove portion in a direction orthogonal to a direction in which the sub-groove portion extends in the object portion.

In the present specification, the terms "front waist region," "back waist region," and "crotch region" are used in a case where the absorbent article is a disposable diaper, and the meanings are as follows. In a pull-on disposable diaper, the front waist region means a region interposed between a pair of joining portions at both lateral end portions that join a front body and a back body in the front body, and the back waist region means a region interposed between the pair of joining portions in the back body. The crotch region means a region between the front waist region and the back waist region. The crotch region also corresponds to a region interposed between the pair of leg openings. In a tape-type disposable diaper, the waist region and the crotch region are partitioned in a fixed state in which the tip ends of a pair of tape fasteners are fixed so as to be adjacent to a predetermined fixing region for the tape fastener. Specifically, the waist region is determined based on a pair of overlapping portions in which a waist forming member of the front body and a waist forming member of the back body overlap with each other in the absorbent article in the above fixed state. The front waist region means a region between the pair of overlapping portions in the front body of the absorbent article. Similarly, the back waist region means a region between the pair of overlapping portions in the back body of the absorbent article. The crotch region means a region between the front waist region and the back waist region.

### <Absorbent article>

### (First embodiment)

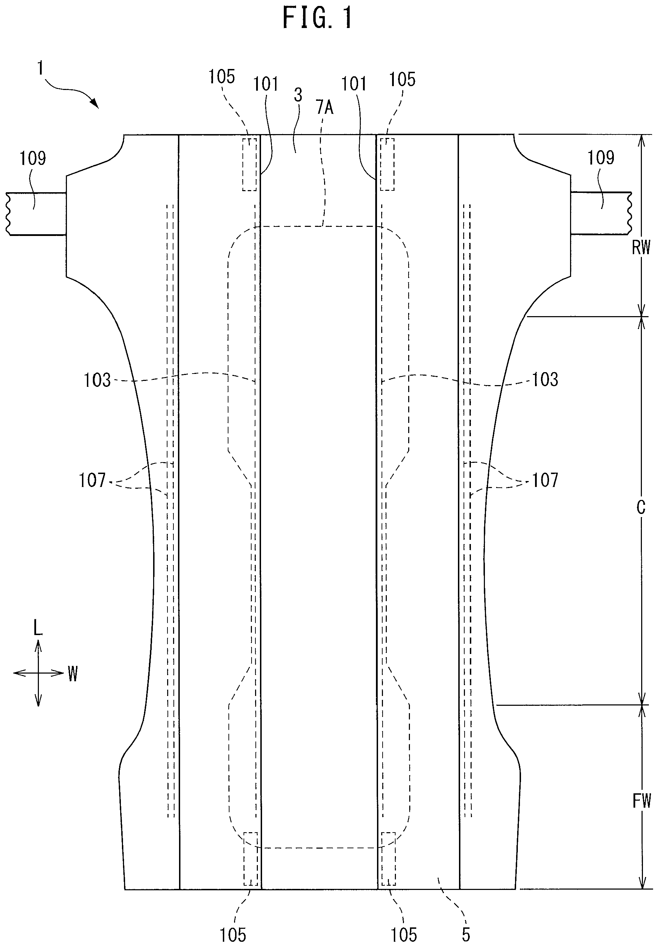

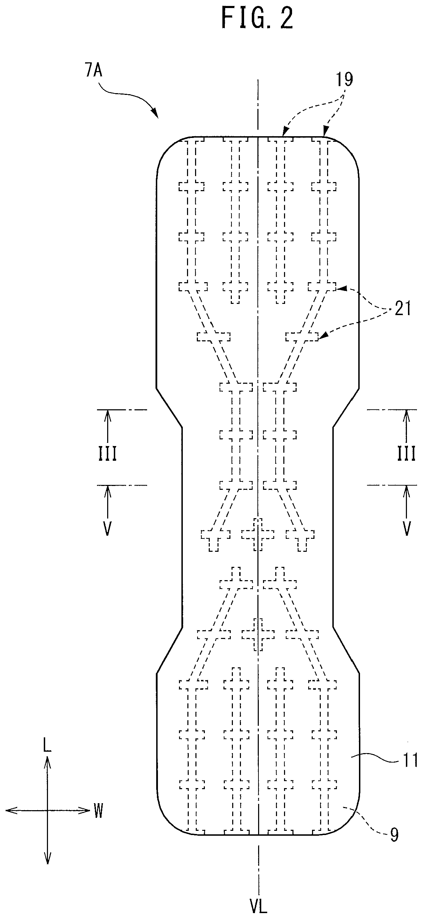

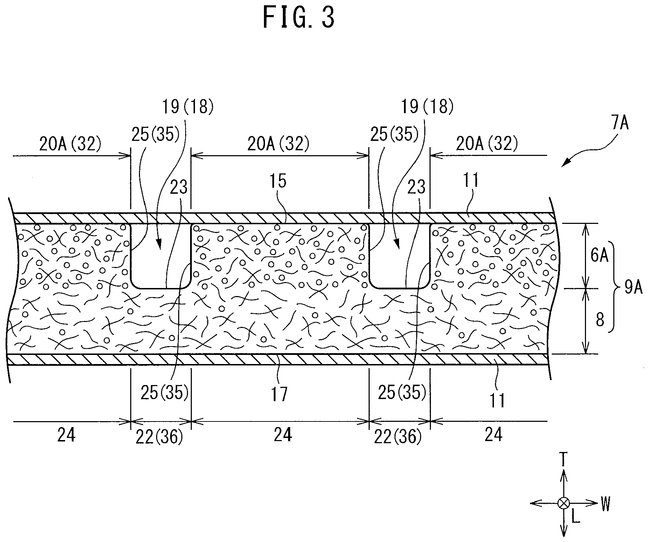

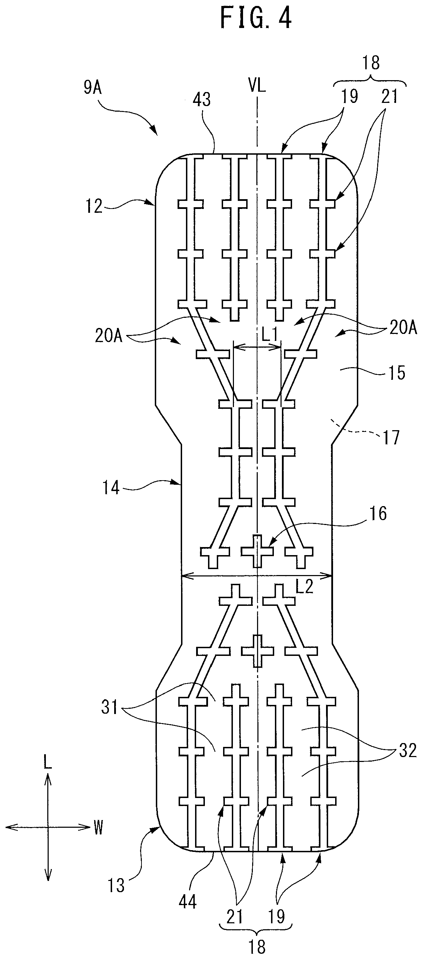

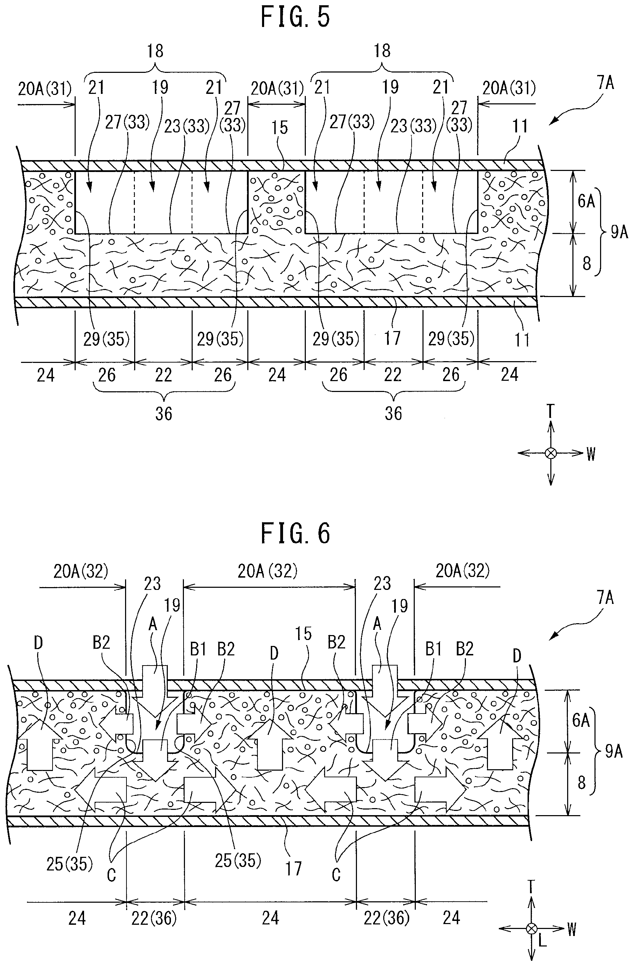

FIGS. 1 to 6 are views showing a configuration example of an absorbent article 1 according to a first embodiment, specifically, a disposable diaper. FIG. 1 is a plan view of the absorbent article according to the first embodiment. FIG. 2 is a plan view of an absorbent body of the absorbent article according to the first embodiment. FIG. 3 is a cross-sectional view taken along a line III-III in FIG. 2 according to the first embodiment. FIG. 4 is a plan view of an absorbent core of the absorbent article according to the first embodiment. FIG. 5 is a cross-sectional view of the absorbent body taken along a line V-V in FIG. 2 according to the first embodiment. FIG. 6 is a schematic view showing the flow of a bodily fluid in a cross section of the absorbent body taken along a line III-III in FIG. 2 according to the first embodiment.

As shown in FIG. 1, the absorbent article 1 includes a liquid-permeable sheet 3, a liquid-impermeable sheet 5, and an absorbent body 7A that is arranged between the liquid-permeable sheet 3 and the liquid-impermeable sheet 5. In a lengthwise direction L, the absorbent article 1 is divided into three regions: a front waist region FW, a back waist region RW, and a crotch region C between the front waist region FW and the back waist region RW. The absorbent body 7A is arranged across the three regions. The absorbent article 1 further includes a pair of leakproof walls 101 including an elastic member 103, a fixed portion 105 that fixes the leakproof wall 101 to the liquid-permeable sheet 3, an elastic member 107 arranged around the leg portion, a tape fastener 109, and the like. It should be noted that these are well-known in the art and will not be described here.

As shown in FIGS. 2 and 3, the absorbent body 7A has a lengthwise direction L, a lateral direction W, and a thickness direction T. The absorbent body 7A includes an absorbent core 9A having a skin facing surface 15 and a non-skin facing surface 17, and a core wrap 11 formed of a tissue that covers the skin facing surface 15 and the non-skin facing surface 17. It should be noted that, in the present embodiment, since the lengthwise direction, the lateral direction, and the thickness direction of the absorbent article 1 are the same as the directions of the absorbent body 7A, the lengthwise direction L, the lateral direction W, and the thickness direction T are also used as the directions of the absorbent article 1. As the core wrap 11, a hydrophilic nonwoven fabric may be used.

The absorbent core 9A includes the skin side layer 6A on the skin facing surface side and the non-skin side layer 8 on the non-skin facing surface side. The skin side layer 6A includes a plurality of groove portions 18 that penetrate the skin side layer 6A in the thickness direction T, and a base potion 20A that is located between the plurality of groove portions 18. As shown in FIG. 4, the absorbent core 9A has a first end portion 12 and a second end portion 13 that are arranged at both end portions in the lengthwise direction L and have the same length in the lateral direction W, and a constricted portion 14 that is arranged at a central part in the lengthwise direction L and has a length in the lateral direction W shorter than the lengths of the first end portion 12 and the second end portion 13. The first end portion 12 and the constricted portion 14, and the second end portion 13 and the constricted portion 14 are respectively connected to each other through inclined sides inclined toward the inner side in the lateral direction W toward the constricted portion 14. The groove portion 18 and the base potion 20A have a shape extending along the lengthwise direction L and alternately extend in the lateral direction W. The groove portions 18 are arranged so as to be line-symmetric with respect to a central axis line VL that bisects the absorbent core 9A in the lateral direction W.

The groove portions 18 have a plurality of main groove portions 19 and a plurality of sub-groove portions 21. The plurality of main groove portions 19 are respectively recessed in the thickness direction T of the absorbent core 9A from the skin facing surface 15 toward the non-skin facing surface 17, and extend in the lengthwise direction L. In the case of the first embodiment, four main groove portions 19 are evenly arranged in the lateral direction W with an end 43 of the first end portion 12 and an end 44 of the second end portion 13 serving as base ends. Among the four main groove portions 19 at the first end portion 12 and the second end portion 13, two inner main groove portions have leading ends respectively towards the first end portion 12 and towards the second end portion 13 with respect to the constricted portion 14.

The two main groove portions 19 located on the outer sides of the second end portion 13 in the lateral direction W extend to be inclined toward the inner side in the lateral direction W toward the constricted portion 14, and have leading ends at positions along the lengthwise direction L. The two main groove portions 19 located on the outer sides of the first end portion 12 in the lateral direction W extend to be inclined toward the inner side in the lateral direction W toward the constricted portion 14, and extend along the lengthwise direction L again in the constricted portion 14. Further, the two main groove portions 19 are inclined toward the outer side in the lateral direction W so as to avoid the leading ends of the two main groove portions 19 that extend from the second end portion 13 and have leading ends at positions along the lengthwise direction L. A substantially cross-shaped cross groove portion 16 is arranged between the leading ends of the two main groove portions 19, that is, at the center of the absorbent core 9A in the lateral direction W.

It is preferable that a space between the main groove portions 19 in the lateral direction W is 15% or more and less than 45% of the minimum length of the absorbent core 9A the lateral direction W in an excretion region. The space between the main groove portions 19 is the length between the centers of the two main groove portions 19. In the case of the first embodiment, the space between the main groove portions 19 in the lateral direction W is a space L1 between the two main groove portions 19 that extend from the first end portion 12 in the constricted portion 14. The minimum length of the absorbent core 9A in the lateral direction W is a lateral length L2 of the constricted portion 14. Therefore, it is preferable that the length L1 is 15% or more and less than 45% of the length L2.

As shown in FIG. 3, the main groove portion 19 includes a main groove bottom face 23 that comes into contact with the non-skin side layer 8, and a main groove side surface 25 that is in contact with the base potion 20A on the outer side of the main groove bottom face 23 in the lateral direction W. The main groove bottom face 23 is the skin side surface of the non-skin side layer 8. The main groove portion 19 has a width that is preferably 0.5 to 3.0 times, more preferably 0.8 to 2.5 times, and even more preferably 1.0 to 2.0 times as much of the thickness of the absorbent body 7A. When the width of the main groove portion 19 is within the above range, the main groove portion 19 easily maintains its water flow function after the absorbent article 1 absorbs the bodily fluid.

In the present specification, unless otherwise specified, the thickness (mm) of an object (for example, an absorbent body or an absorbent core) is measured as follows. FS-60DS (the measurement surface: 44 mm (in diameter), the measurement pressure: 3 g/cm²), manufactured by Daiei Kagaku Seiki MFG, Co., Ltd., is prepared, five different portions of the object are applied with pressure under the standard condition (the temperature: 23±2°C, the relative humidity: 50±5%), the thickness at each of the portions after 10 seconds from the pressure application is measured, and the average value of the five measurement values is used as the thickness of the absorbent body. It should be noted that in the absorptiveness test described below, also in a case of measuring the thickness of the absorbent article after the second cycle, measurement is performed in the same manner.

As shown in FIG. 4, the base end of each of the plurality of sub-groove portions 21 communicates with the main groove portion 19, and the leading end extends in the lateral direction W and has a leading end. In the case of the first embodiment, the sub-groove portion 21 extends along the lateral direction W from the first end portion 12 to the constricted portion 14 and from the second end portion 13 to the constricted portion 14 even in a range where the main groove portion 19 is inclined. As shown in FIG. 5, the sub-groove portion 21 is recessed in the thickness direction T of the absorbent core 9A from the skin facing surface 15 toward the non-skin facing surface 17and extends in the lateral direction W. The depth of the sub-groove portion 21 is the same as the depth of the main groove portion 19. The sub-groove portion 21 has a sub-groove bottom face 27 that is in contact with the non-skin side layer 8 and a sub-groove side surface 29 that is in contact with the base potion 20A on the outer side of the sub-groove bottom face 27 in the lateral direction W. The sub-groove bottom face 27 is the skin side surface of the non-skin side layer 8. Usually, the sub-groove portions 21 are formed on both sides of the main groove portion 19. However, in the main groove portions 19 at two ends located on the outermost side of the absorbent core 9A in the lateral direction W, the sub-groove portions 21 on the outer sides of the main groove portions 19 can be omitted.

In the present specification, in a case where the main groove bottom face 23 and the sub-groove bottom face 27 are not particularly distinguished from each other, the main groove bottom face 23 and the sub-groove bottom face 27 will be referred to as a groove bottom face 33. The opening of the groove portion 18 that faces the groove bottom face 33 is blocked with the core wrap 11.

As shown in FIGS. 3 and 5, the base potion 20A has a narrow portion 31 located between facing sub-groove side surfaces 29 that extend from the main groove portions 19 located on both sides of the base potion 20A toward the base potion 20A, and a wide portion 32 located between the main groove side surfaces 25. A top surface 34 of the base potion 20A is the skin facing surface 15. The top surface 34 comes into contact with the main groove side surface 25 and the sub-groove side surface 29, respectively, on the outer side in the lateral direction W. The main groove side surface 25 and the sub-groove side surface 29 also serve as base side surfaces 35. The sub-groove portions 21 that form the narrow portion 31 need to face each other, but the sub-groove portion 21 on the opposite side to the narrow portion 31 does not have to extend from the same position of the main groove portion 19. Each of the plurality of base portions 20A is continuous from the first end portion 12 to the second end portion 13 through the constricted portion 14. The plurality of base portions 20A that are adjacent to each other through the groove portions 18 are continuous between the leading ends of the main groove portions 19 in the constricted portion 14 (FIG. 4).

As shown in FIG. 5, the non-skin side layer 8 includes, in the thickness direction T, a plurality of main groove portion corresponding portions 22 at positions that overlap the plurality of main groove portions 19, a plurality of sub-groove portion corresponding portions 26 at positions that overlap the plurality of sub-groove portions 21, and a plurality of base corresponding portions 24 at positions that overlap the plurality of base portions 20A. In a case where the main groove portion corresponding portion 22 and the sub-groove portion corresponding portion 26 are not particularly distinguished from each other, the main groove portion corresponding portion 22 and the sub-groove portion corresponding portion 26 will be referred to as a groove portion corresponding portion 36. The non-skin facing surface 17 of the non-skin side layer 8 is flat. The plurality of base portions 20A, the plurality of groove portion corresponding portions 36, and the plurality of base corresponding portions 24 may be integrally formed.

The thickness of both the skin side layer 6A and the non-skin side layer 8 is preferably 0.1 to 5 mm, more preferably 0.5 to 4 mm, and even more preferably 1 to 3 mm. It should be noted that the thickness of each of the skin side layer 6A and the non-skin side layer 8 can be measured in the following non-contact method using the laser displacement meter (for example, high-precision two-dimensional laser displacement meter LJ-G series (model: LJ-G030) manufactured by Keyence Corporation). The absorbent core 9A which is obtained by blowing a cold spray onto the absorbent article 1 so as to peel off the liquid-permeable sheet 3, the liquid-impermeable sheet 5, and the core wrap 11, is cut into a size of 100 mm × 100 mm, and is used as a sample. The sample is placed on a horizontal measurement table in a manner such that the skin facing surface 15 having the plurality of groove portions 18 and the plurality of base portions 20A formed thereon faces upward, the displacement from the measurement table is measured by the laser displacement meter for five different base portions 20A, and the average value of the five measurement values is set as Ax (mm). Similarly, the displacement from the measurement table is measured by the laser displacement meter for five different groove portions 18 (main groove portions 19), and the average value of the five measurement values is set as Ay (mm). The thickness of the non-skin side layer 8 is Ay (mm), and the thickness of the skin side layer 6A is calculated from the difference between Ax (mm) and Ay (mm).

The absorbent core 9A includes water-absorbent fibers 30 and superabsorbent polymer particles (SAP) 32, and has a function of absorbing and holding the bodily fluid discharged to the absorbent article 1. A ratio of the superabsorbent polymer particles contained in the non-skin side layer (main groove portion corresponding portions 22, base corresponding portions 24) 8 to the superabsorbent polymer particles contained in the entire absorbent core 9A is 0% or more and less than 50%, preferably 0% or more and less than 30%, and more preferably 0% or more and 20% or less.

In the first embodiment, the average basis weight of the water-absorbent fibers in both the skin side layer 6A and the non-skin side layer 8 is preferably 50 to 250 g/m², and more preferably 80 to 200 g/m². The basis weight is measured according to the following measurement method. The sheet is cut into a size of 5 cm × 5 cm and is used as a sample. The mass is measured after a drying treatment in an atmosphere of 100°C or higher. Next, the measured mass is divided by the area of the sample to calculate the basis weight of the sample. The value obtained by averaging the basis weights of 10 samples is defined as the basis weight of the sheet. The average basis weight of the superabsorbent polymer particles in the non-skin side layer 8 is preferably 0 to 200 g/m², and more preferably 0 to 150 g/m², and the average basis weight of the superabsorbent polymer particles in the skin side layer 6A is preferably 100 to 500 g/m², and more preferably 150 to 400 g/m².

The average density (hereinafter, referred to as "first average density") of the superabsorbent polymer particles in the non-skin side layer (main groove portion corresponding portions 22, base corresponding portions 24) 8 is smaller than the average density (hereinafter, referred to as "second average density") of the superabsorbent polymer particles in the skin side layer (base portions 20A) 6A. In the case of the first embodiment, the first average density is, for example, 0 to 0.15 g/cm³, 0 to 0.1 g/cm³, or 0 to 0.08 g/cm³, and the second average density is, for example, 0.03 to 0.4 g/cm³, 0.04 to 0.35 g/cm³, or 0.05 to 0.3 g/cm³.

A method of measuring the average density of the superabsorbent polymer particles is as follows. Five samples having a predetermined length and a predetermined width are cut out from the absorbent core 9A (for example, 4 mm × 4 mm), the superabsorbent polymer particles contained in each sample are selected, the total mass of the superabsorbent polymer particles contained in each sample is measured, and the measurement value is divided by the volume of the sample obtained by the thickness of the sample and the area of the sample to obtain the average value.

In the present specification, in a case of evaluating the average density of the superabsorbent polymer particles in each of the portions of the base potion 20A and the non-skin side layer (main groove portion corresponding portion 22, base corresponding portion 24) 8 in the absorbent core 9A, the following method may be employed. For example, the absorbent article 1 serving as a sample is impregnated with liquid nitrogen so as to be frozen, and then, the absorbent article 1 is cut with a razor in the thickness direction T, so as to obtain a cross section at the surface crossing the direction in which the main groove portion 19 extends. Next, the temperature of the sample is returned to room temperature, and a cross-sectional image with a magnification of 50 times is obtained by using an electron microscope (for example, VE7800 manufactured by Keyence Corporation). In the cross-sectional image, the degrees of the average density of the superabsorbent polymer particles at each of the portions of the base potion 20A, the main groove portion corresponding portion 22, and the base corresponding portion 24 is visually evaluated. It should be noted that, in a case of evaluating the degrees of the average density of the superabsorbent polymer particles in the sub-groove portion corresponding portion 26 and the average density of the superabsorbent polymer particles in each of the base potion 20A and the base corresponding portion 24, evaluation can be performed using a cross-sectional image in the surface crossing the direction in which the sub-groove portion 21 extends in the same method as described above, instead of the above method.

Since the absorbent article 1 has the groove portions 18 in the skin side layer 6A and the average density of the superabsorbent polymer particles is in the relationship of the non-skin side layer 8 < the skin side layer 6A, the bodily fluid moves in the absorbent core 9A as shown in FIG. 6, for example.
(i) The bodily fluid that has reached the core wrap 11 moves to the groove portion 18 by gravity (arrow A).
(ii) The bodily fluid that has moved to the groove portion 18 is transported in the lengthwise direction L and permeates into the groove portion corresponding portion 36 and the base potion 20A (arrows B 1 and B2).
(iii) The bodily fluid that has permeated into the groove portion corresponding portion 36 is diffused in the lengthwise direction L and the lateral direction W through the base corresponding portion 24 (arrow C).
(iV) The bodily fluid that has been diffused in the base corresponding portion 24 can be sucked up to the base potion 20A containing a large amount of superabsorbent polymer particles and having a high density and held in the superabsorbent polymer particles (arrow D).

Since the absorbent article 1 includes the groove portion 18 and the base potion 20A in which the density of the superabsorbent polymer particles is high on the skin side layer 6A, and the water absorption rate of the superabsorbent polymer particles is not high, most of the bodily fluid that has reached the core wrap 11 is immediately passed to the groove portion 18. The bodily fluid that has passed to the groove portion 18 is transported in the lengthwise direction L of the absorbent core 9A through the main groove portion 19, and is absorbed into the inside of the absorbent core 9A from the main groove portion 19 and the sub-groove portion 21 communicating with the main groove portion 19. That is, the bodily fluid permeates from the main groove bottom face 23 to the main groove portion corresponding portion 22, and from the sub-groove bottom face 27 to the sub-groove portion corresponding portion 26, and also permeates from the main groove side surface 25 to the wide portion 32, and from the sub-groove side surface 29 to the narrow portion 31. Further, while the bodily fluid that has permeated into the main groove portion corresponding portion 22 and the sub-groove portion corresponding portion 26 is diffused through the base corresponding portion 24, the bodily fluid can be sucked up to the base potion 20A containing a large amount of superabsorbent polymer particles and having a high density, and held in the superabsorbent polymer particles. Therefore, even when the bodily fluid is repeatedly absorbed, the absorbent article 1 can maintain the absorption performance, for example, absorption rate by repeating the above-described cycle.

The first average density of the superabsorbent polymer particles contained in the groove portion corresponding portion 36 and the base corresponding portion 24 is lower than the second average density of the superabsorbent polymer particles contained in the base potion 20A. The absorption rate of the superabsorbent polymer particles is low, and the water absorption and swelling of the superabsorbent polymer particles, which are blended in the fiber matrix and compressed at a high density, is further suppressed. Therefore, since the swelling of the groove portion corresponding portion 36 is suppressed, the bodily fluid in the groove portion 18 permeates more into the groove portion corresponding portion 36. Further, since the swelling of the base corresponding portion 24 is suppressed, the bodily fluid absorbed by the groove portion corresponding portion 36 is diffused in the lengthwise direction L through the groove portion corresponding portion 36. While the bodily fluid is diffused in the lengthwise direction L, part of the bodily fluid is transferred to the base corresponding portion 24. The bodily fluid that has transferred to the base corresponding portion 24 can be sucked up to the base potion 20A and held in the base potion 20A. Therefore, in the absorbent article 1, when the diffusion rate is increased with respect to the sucking rate of the base potion 20A by suppressing the swelling of the groove portion corresponding portion 36 and the base corresponding portion 24, the superabsorbent polymer particles of the base potion 20A can be prevented from swelling and becoming excessively large. That is, in the absorbent article 1, when the bodily fluid is diffused in a wider range, a local increase in the thickness can be suppressed. As a result, in the absorbent article 1, the entire thickness is prevented from increasing even when the bodily fluid is repeatedly absorbed, and a feeling of wearing can be maintained.

The ratio of the superabsorbent polymer particles contained in the non-skin side layer 8 to the superabsorbent polymer particles contained in the entire absorbent core 9A is 0% or more and less than 30%. In the case of the above-described range, since the superabsorbent polymer particles contained in the plurality of groove portion corresponding portions 36 and the plurality of base corresponding portions 24 can be sufficiently reduced compared with the base potion 20A, the swelling of the groove portion corresponding portions 36 and the base corresponding portions 24 can be suppressed. Therefore, in the absorbent article 1, an increase in the thickness can be more reliably suppressed. Further, in the absorbent article 1, the bodily fluid can be more rapidly diffused in the lengthwise direction L and the lateral direction W through the groove portion corresponding portion 36 and the base corresponding portion 24.

The space L1 between the main groove portions 19 in the lateral direction W is 15% or more and less than 45% of the minimum length L2 of the absorbent core 9A in the lateral direction W in the excretion region. The absorbent article 1 can prevent the bodily fluid from concentrating at the center of the absorbent core 9A, and can disperse the bodily fluid and more rapidly absorb the bodily fluid from the groove portion 18. Since the plurality of base portions 20A, the plurality of groove portion corresponding portions 36, and the plurality of base corresponding portions 24 are integrally formed, the bodily fluid can be more smoothly transferred between the groove portion corresponding portions 36, the base corresponding portions 24, and the base portions 20A, and thus the bodily fluid can be more rapidly diffused.

Description has been made on a case where the average density of the superabsorbent polymer particles in the sub-groove portion corresponding portion 26 is the first average density that is lower than the second average density. However, the present invention is not limited thereto, and the average density may be higher than the first average density and equal to or lower than the second average density. Even in a case where the average density of the superabsorbent polymer particles in the sub-groove portion corresponding portion 26 is higher than the first average density, the non-skin side layer 8 has the main groove portion corresponding portion 22 in addition to the base corresponding portion 24, and thus the same effect as in the above embodiment can be obtained.

### (Second embodiment)

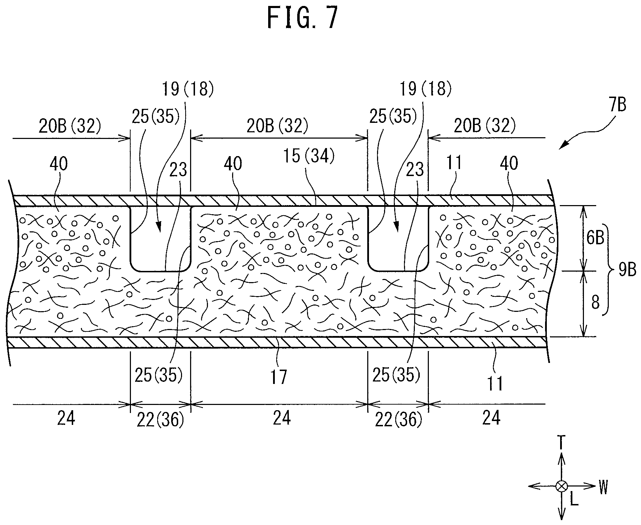

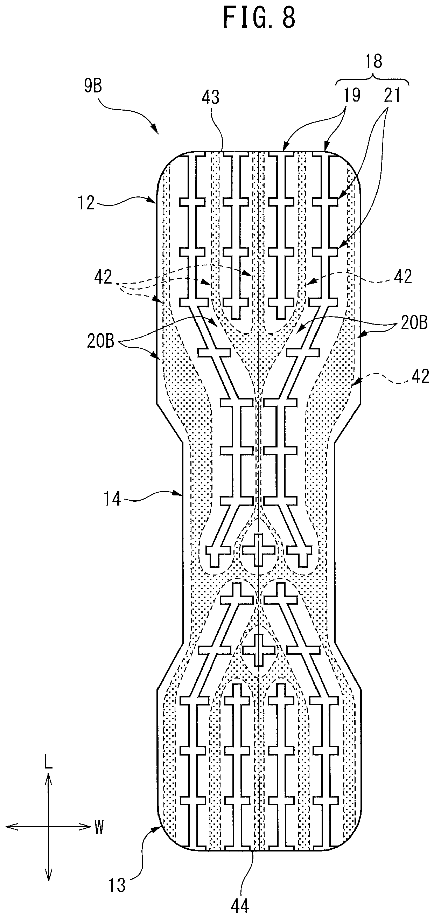

Hereinafter, an absorbent article according to a second embodiment will be described. The configurations similar to those of the first embodiment are denoted by similar reference signs, and the description thereof will be omitted. FIG. 7 is a cross-sectional view taken along a line III-III in FIG. 2. FIG. 8 is a plan view of an absorbent core 9B of the absorbent article 1.

An absorbent body 7B includes the absorbent core 9B and the core wrap 11. The absorbent core 9B is formed of a skin side layer 6B and a non-skin side layer 8, and the skin side layer 6B includes a plurality of groove portions 18 and a plurality of base portions 20B. Each of the base portions 20B includes a surface layer region 40 and a central region 42. The surface layer region 40 includes the top surface 34 and the base side surface 35 and has a constant thickness from the top surface 34 and the base side surface 35 into the base potion 20B. The thickness of the surface layer region 40 is, for example, in a range of 1/5 of the length of the base potion 20B in the lateral direction W. The average density (hereinafter, referred to as "third average density") of the superabsorbent polymer particles contained in the surface layer region 40 is lower than the second average density. The third average density is approximately 30% of the second average density.

The central region 42 includes the centers of the plurality of base portions 20B in the lateral direction W, and is in contact with the surface layer region 40. The base side surface 35 side and the top surface 34 side of the central region 42 is in contact with the surface layer region 40. The central region 42 is continuous from the end 43 of the first end portion 12 to the end 44 of the second end portion 13 through the constricted portion 14. The length of the central region 42 in the lateral direction W is, for example, in a range of 3/5 of the length of the base potion 20B in the lateral direction W. The average density (hereinafter, referred to as "fourth average density") of the superabsorbent polymer particles contained in the central region 42 is higher than the second average density. The fourth average density is approximately 150% of the second average density. The presence of the surface layer region 40 and the central region 42 can be confirmed by analyzing the distribution of the superabsorbent polymer particles from the X-ray image of the absorbent core.

Since the third average density of the surface layer region 40 is lower than the second average density, the bodily fluid that has passed to the groove portion 18 is transported in the lengthwise direction L of the absorbent core 9B through the main groove portion 19, infiltrated into the surface layer region 40 in which the average density of the superabsorbent polymer particles is lower, and thus more rapidly permeates into the base potion 20B. That is, the bodily fluid more rapidly permeates from the main groove side surface 25 to the wide portion 32, and from the sub-groove side surface 29 to the narrow portion 31. Therefore, the absorbent article 1 can rapidly absorb the bodily fluid in the groove portion 18. Further, the surface layer region 40 in which there are few superabsorbent polymer particles is less likely to cause a phenomenon that the superabsorbent polymer particles that have absorbed water and swollen spill into the groove portion 18 and block the groove portion 18. Therefore, the absorbent article 1 can maintain a rapid absorption rate even when the bodily fluid is repeatedly absorbed.

Since the fourth average density of the central region 42 is higher than the second average density, the swelling of the base side surface 35 that is in contact with the groove portion 18 can be suppressed and the collapse of the groove portion 18 can be suppressed. Therefore, in the absorbent article 1, even in a case where the bodily fluid is repeatedly absorbed, the bodily fluid can more reliably permeate from the groove portion 18 to the groove portion corresponding portion 36 and be absorbed.

Since the central region 42 is continuous from the end 43 of the first end portion 12 to the end 44 of the second end portion 13 of the absorbent core 9B, the swelling of the base side surface 35 that is in contact with the groove portion 18 can be suppressed across the entire region of the absorbent core 9B in the lengthwise direction L. Therefore, in the absorbent article 1, the collapse of the groove portion 18 can be suppressed, and the bodily fluid can be continuously and repeatedly diffused in the lengthwise direction L.

### <Modified example>

The present invention is not limited to the above-described embodiments and these can be appropriately combined, modified, or the like without departing from the scope of the present invention.

### <Method of Manufacturing Absorbent Article>

In a case of manufacturing the absorbent article 1 having the configuration described in the above embodiment, there is no limitation on the manufacturing method. However, for example, the following method can be used. Incidentally, in the present specification, the "the machine direction of a material or a product" is referred to as "the MD direction", "the direction orthogonal to the MD direction on a horizontal plane" (that is, the width direction of the manufacturing line) is referred to as "the CD direction", and "the direction orthogonal to the MD direction and the CD direction" (that is, the vertical direction of the manufacturing line) is referred to as "the TD direction".

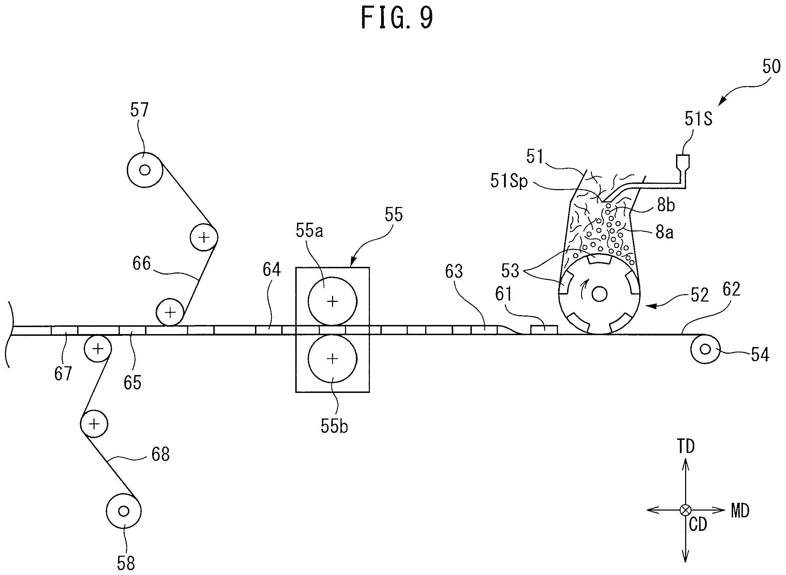

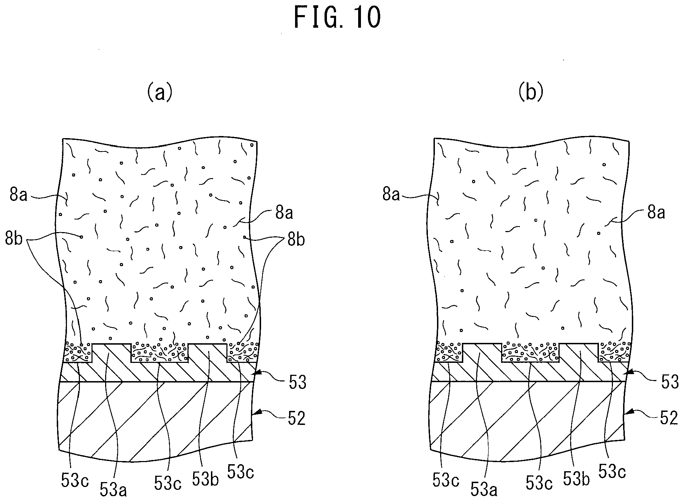

FIG. 9 is a schematic view showing a configuration example of a manufacturing apparatus 50 for manufacturing the absorbent article 1 according to the embodiment. In addition, FIGS. 10(a) and 10(b) are schematic views showing a state in which the absorbent material is supplied on a suction drum 52 of the manufacturing apparatus 50 in FIG. 9.

The manufacturing apparatus 50 includes a transport duct 51 and a suction drum 52. The transport duct 51 transports the absorbent material that includes an opened water-absorbent fibers 8a and superabsorbent polymer particles 8b to the suction drum 52. The transport duct 51 includes a transport duct nozzle 51S that discharges the superabsorbent polymer particles 8b from a discharge port 51Sp to the suction drum 52. The suction drum 52 is rotatable, and sucks the absorbent material in the transport duct 51 and overlays the absorbent material on a plurality of concave mold members 53 arranged with a certain space along the circumferential direction of the outer circumferential surface to form a first laminated body 61 which becomes the absorbent core 9A of the absorbent body 7A in the later process.

The manufacturing apparatus 50 further includes an unwinding roll 54 for a core wrap continuous body. The unwinding roll 54 unwinds a long core wrap continuous bodies 62 toward the suction drum 52. The suction drum 52 places the first laminated body 61 on the outer circumferential surface of the suction drum on the core wrap continuous body 62. The first laminated body 61 placed on the core wrap continuous body 62 is covered with the core wrap continuous body 62 to form a second laminated body 63. The manufacturing apparatus 50 further includes a pressing device 55. The pressing device 55 includes a pair of press rolls 55a and 55b that apply pressure and compress the second laminated body 63 in the thickness direction (TD direction). A third laminated body 64 is formed by pressing the second laminated body 63. The manufacturing apparatus 50 further includes an unwinding roll 57 for a liquid-permeable sheet continuous body. The unwinding roll 57 unwinds and laminates a long liquid-permeable sheet continuous body 66 that serves as the liquid-permeable sheet 3 on one surface (in the case of FIG. 10, the upper surface) of the third laminated body 64. The liquid-permeable-sheet continuous body 66 is laminated on the third laminated body 64 to form a fourth laminated body 65. The manufacturing apparatus 50 further includes an unwinding roll 58 for a liquid-impermeable sheet continuous body. The unwinding roll 58 unwinds and joins a long liquid-impermeable sheet continuous body 68 that serves as the liquid-impermeable sheet 5 to the surface (in the case of FIG. 10, the lower surface) of the fourth laminated body 65 opposite to the liquid-permeable sheet continuous body 66. The liquid-impermeable sheet continuous body 68 is laminated on the fourth laminated body 65 to form a fifth laminated body 67.

It should be noted that the manufacturing apparatus 50 includes a device (not shown) that cuts the fifth laminated body 67 into the shape of the absorbent article 1 as a product so as to be a single absorbent article 1, and each of the devices that compresses leakproof walls 101 and the tape fasteners 109 onto the fifth laminated body 67 on the downstream side in the MD direction with respect to the unwinding roll 58. However, since these devices are ordinary devices known in this technical field, the detailed explanation is omitted.

In a case where the absorbent article 1 is manufactured by using the manufacturing apparatus 50 described above, the following steps are performed. That is, the following steps are sequentially performed: a first step of forming the first laminated body 61; a second step of covering the first laminated body 61 with the core wrap continuous body 62 to form the second laminated body 63; and a third step of compressing the second laminated body 63 in the TD direction with the pressing device 55 to form the third laminated body 64. Further, a fourth step of overlaying the liquid-permeable sheet continuous body 66 on the third laminated body 64 to form the fourth laminated body 65, and a fifth step of joining the liquid-impermeable sheet continuous body 68 to the fourth laminated body 65 to form the fifth laminated body 67 are sequentially performed.

First, the first step of forming the first laminated body 61 that will ultimately constitute the absorbent core 9A of the absorbent body 7A is performed. In the first step, the absorbent material which includes the water-absorbent fibers 8a and the superabsorbent polymer particles 8b is sucked by the suction drum 52 through the transport duct 51, and the absorbent material is laminated in the mold member 53 on the outer circumferential surface of the suction drum 52 to form the first laminated body 61.

Here, the mold member 53 includes four pairs of protruding portions (in FIG. 10, only 53a and 53b are shown) having a quadrangular cross section which extend in the circumferential direction of the suction drum 52 in a bottom portion 53c. Specifically, each of the protruding portions 53a and 53b has a rectangular shape. These protruding portions 53a and 53b are arranged at positions adapted to the positions of the groove portions 18 of the absorbent body 7A so as to have the shape and the width in the lengthwise direction adapted to the shape and the width in the lengthwise direction of the groove portions 18.

FIG. 10(a) shows a state in which the absorbent material is supplied on the suction drum 52 in the first region (for example, the first 1/5 of the entire region) and in an intermediate region (for example, 1/5 of the middle area of the entire region) of the region in which the absorbent material is supplied in the transport duct 51. This region is a region controlled so that the superabsorbent polymer particles 8b discharged from the discharge port 51Sp are supplied by a predetermined amount and the water-absorbent fibers 8a are supplied by a predetermined amount. Therefore, the superabsorbent polymer particles 8b and the water-absorbent fibers 8a are supplied and overlaid on each other. The total laminate thickness of the superabsorbent polymer particles 8b and the water-absorbent fibers 8a is the same as the height of the protruding portions 53a and 53b. In this manner, the skin side layer 6A is formed.

FIG. 10(b) shows a state in which the absorbent material is supplied on the suction drum 52 in the last region (for example, the last 1/5 of the entire region) of the region in which the absorbent material is supplied in the transport duct 51. This region is a region controlled so that the superabsorbent polymer particles 8b discharged from the discharge port 51Sp are supplied by a small amount (less than the "predetermined amount" of (a)) or almost no amount of the superabsorbent polymer particles are supplied and the water-absorbent fibers 8a are supplied by a predetermined amount. Therefore, mainly the water-absorbent fibers 8a are supplied and overlaid on each other. The total laminate thickness of the superabsorbent polymer particles 8b and the water-absorbent fibers 8a is larger than the height of the protruding portions 53a and 53b. Therefore, the non-skin side layer 8 is further formed. The absorbent core 9A according to the first embodiment can be finally obtained by removing the absorbent core 9A from the suction drum 52 and turning the absorbent core upside down in the drawing.

In a case of forming the base potion 20B having the surface layer region 40 and the central region 42 according to the second embodiment, before the state of FIG. 10(a), the superabsorbent polymer particles 8b discharged from the discharge port 51Sp are supplied by a small amount (less than the "predetermined amount of (a)) or almost no amount of the superabsorbent polymer particles are supplied and the water-absorbent fibers 8a are supplied by a predetermined amount. Then, the surface layer region 40 can be formed on the top surface 34 side. Thereafter, when the superabsorbent polymer particles 8b and the water-absorbent fibers 8a are overlaid in the order of FIGS. 10(a) and 10(b), the absorbent material is supplied from the transport duct nozzle (not shown) that discharges the superabsorbent polymer particles 8b in a relatively large amount. In this case, since the superabsorbent polymer particles 8b are contained in a high discharge amount, when the superabsorbent polymer particles 8b collide with the protruding portions 53a and 53b, the superabsorbent polymer particles 8b easily rebound, and the surface layer region 40 having a low average density of the superabsorbent polymer particles 8b is formed in the vicinity of the protruding portions 53a and 53b. At the same time, the protruding portions 53a and 53b of the superabsorbent polymer particles are collected at the center, and the central region 42 in which the average density of the superabsorbent polymer particles is high is formed at the center. As described above, the base potion 20B having the surface layer region 40 and the central region 42 is formed, and thus the absorbent core 9B according to the second embodiment can be obtained.

In the second step, the rotated suction drum 52 transfers the first laminated body 61 in the mold member 53 onto the core wrap continuous body 62 (coated with an adhesive) that is unwound from the unwinding roll 54 for the core wrap continuous body and is moved in the MD direction. Then, the core wrap continuous body 62 is folded along the outer circumferential surface of the first laminated body 61 in the CD direction orthogonal to the MD direction with folding means (not shown), and the core wrap continuous body 62 is covered with the first laminated body 61 by being wound to form the long second laminated body 63. Next, in the third step, the second laminated body 63 is passed between the pair of press rolls 55a and 55b of the pressing device 55 to compress the second laminated body 63 in the TD direction. At this time, the third laminated body 64 is formed. Next, in the fourth step, the liquid-permeable continuous body 66 which is unwound from the unwinding roll 57 for the liquid-permeable continuous body is laminated on the upper surface of the third laminated body 64 with an adhesive agent such as a hot-melt adhesive agent to form the long fourth laminated body 65. Next, in the fifth step, the liquid-impermeable sheet continuous body 68 which is unwound from the unwinding roll 58 for the liquid-impermeable sheet continuous body is joined to the lower surface of the fourth laminated body 65 with an adhesive such as a hot-melt adhesive to form the long fifth laminated body 67. After the fifth step is completed, the fifth laminated body 67 is cut into the shape of the absorbent article 1 by a cutting device. Due to that, the absorbent article 1 is completed. Each step can be appropriately changed without departing from the spirit of the present disclosure.

### Examples

Hereinafter, the present invention will be described by showing examples, but the present invention is not limited to these examples.

### (a) Sample

An absorbent core having a size of 335 mm × 100 mm (lengthwise direction × lateral direction) and containing pulp fibers and superabsorbent polymer particles (SAP) as water-absorbent fibers was manufactured according to the above-described manufacturing method. In the steps of FIGS. 10(a) and 10(b), the basis weights of the pulp fibers and the superabsorbent polymer particles were appropriately adjusted. The lateral length of the main groove portion was 4 mm, the lateral length of the sub-groove portion was 6 mm, and the lengthwise length of the sub-groove portion was 4 mm.

Next, as for the absorbent core, hydrophilic polypropylene spunbond (PPSB, basis weight: 15 g/m², 355 mm × 100 mm) was used as a core wrap of the skin side layer, and hydrophilic polypropylene spunbond (PPSB, basis weight: 15 g/m², 355 mm × 140 mm) was used as a core wrap on the non-skin layer side with a hot-melt adhesive interposed therebetween. The skin side layer and the non-skin side layer were covered with two core wraps, a side crossing the non-skin side layer in the lateral direction was wound toward the skin side layer to be attached to the surface of the skin side layer, and thus an absorbent body was formed. The thickness of the absorbent core was adjusted by pressing the absorbent body with a hydraulic press machine. Therefore, the absorbent bodies of Examples 1 to 6 were formed from the absorbent cores of Examples 1 to 6, and the absorbent bodies of Reference Example 1 and Comparative Example 1 were formed from the absorbent cores of Reference Example 1 and Comparative Example 1. Thereafter, a liquid-permeable sheet (air-through nonwoven fabric) was attached to the layer side on the skin side of each absorbent body, and a liquid-impermeable sheet (polyethylene film) was attached to the layer side on the non-skin side. Therefore, simple absorbent articles of Examples 1 to 6 were formed from the absorbent bodies of Examples 1 to 6, and simple absorbent articles of Reference Example 1 and Comparative Example 1 were formed from the absorbent bodies of Reference Example 1 and Comparative Example 1. Specific configurations of examples, reference examples, and comparative examples are as shown in Table 1. In Table 1, "Direction of groove portion" indicates a position where the groove portion is formed. A case where the groove portion is formed in the skin side layer is represented as "up" and a case where the groove portion is formed in the non-skin side layer is represented as "down". In Table 1, "SAP composition ratio (%)" indicates the composition ratio of SAP contained in the skin side layer and the non-skin side layer with respect to SAP contained in the entire absorbent core. In Examples 1 to 6, the groove portions are formed in the skin side layer. In Example 1, the skin side layer was formed of pulp fibers and SAP, and the non-skin side layer was formed of only pulp fibers. In Examples 2 to 6, the ratio of SAP in the non-skin side layer was adjusted in a range smaller than the ratio of SAP in the skin side layer. Reference example 1 is different from Example 1 in that the groove portions are formed in the non-skin side layer. Comparative Example 1 is different from Examples 2 to 6 only in that the ratios of SAP contained in the skin side layer and the non-skin side layer are the same. In Examples 1 to 6, Reference Example 1, and Comparative Example 1, the pulp fibers had the basis weight shown in Table 1, and the thicknesses of the skin side layer and the non-skin side layer were set to be the same.

### (B) Evaluation

The absorbent articles of Examples 1 to 6, Reference Example 1, and Comparative Example 1 were subjected to an absorbent test defined below, and the thickness change, absorption rate, and liquid return amount (rewettability) were evaluated. The results of the evaluation are shown in Tables 1 and 2.

### <Absorbent Test>

(1) A cross was marked at a central position A of the absorbent article in the lengthwise direction, and a cross was further marked at a position B 50 mm on the stomach-side of the absorbent article in the lengthwise direction. After measuring the thickness T1 at the central position A, the absorbent article is set in a U-shaped instrument having a substantially U-shaped side view. It should be noted that the absorbent article is set so that the central position of the absorbent body in the lengthwise direction matches the central part of the U-shaped instrument (the position at which the height is the lowest).

### <First Cycle>

(2) 80 mL of artificial urine (first time) is injected from a burette at a rate of 80 mL/10 sec to the position B of the absorbent body.

### <Second Cycle>

(3) After 10 minutes from the start of the injection of the artificial urine of the first time, 80 mL of artificial urine (second time) is injected into the position B of the absorbent body from a burette at a rate of 80 mL/10 sec.
(4) The absorbent article is removed from the U-shaped instrument, the absorbent article is expanded on an acrylic flat plate in a manner such that the liquid-permeable sheet is to be the upper surface, the thickness T2 at the central position A in the lengthwise direction is measured, and a difference in thickness (T2 - T1) ("Thickness change" in Tables 1 and 2) is recorded.

### <Third Cycle>

(5) The absorbent article is returned to the U-shaped instrument, and after 10 minutes from the start of the second injection of artificial urine, 80 mL of artificial urine (third time) is injected from the burette into the position B of the absorbent body at a rate of 80 mL/10 seconds.
(6) The absorption time (240 mL) until the artificial urine disappears from the surface is measured and recorded ("Absorption rate" in Table 1 and 2).
(7) After 4 minutes from the start of the injection of the artificial urine of the third time, the absorbent article is removed from the U-shaped instrument, and the absorbent article is expanded on an acrylic flat plate in a manner such that the liquid-permeable sheet is to be the upper surface, and is left to stand for 1 minute.
(8) After 5 minutes from the start of the injection of the artificial urine of the third time, approximately 60 g of filter paper with a size of 100 mm × 100 mm is placed on the liquid-permeable sheet of the absorbent article with the artificial urine injection point as the center. Further, a weight of 3.5 kg with a size of 100 mm × 100 mm × 50 mm (height) is placed thereon. It should be noted that as for the filter paper, the mass before the test is measured in advance.
(9) After 8 minutes from the start of injection of the artificial urine of the third time, the weight is removed, the mass of the filter paper is measured, the mass of the filter paper before the test is subtracted, and the difference is regarded as the liquid return amount ("liquid return amount" in Tables 1 and 2).

It should be noted that the artificial urine was prepared by dissolving 200 g of urea, 80 g of sodium chloride, 8 g of magnesium sulfate, 3 g of calcium chloride, and approximately 1 g of a dye (blue No.1) in 10 L of ion exchange water.

### [Table 1]

**Table 1**

| No. | Direction of groove portion | Basis weight of pulp fibers | Basis weight of SAP | SAP composition ratio (%) | | Thickness change (160 ml) | Absorption rate (240 ml) | Liquid return amount (240 ml) |
|---|---|---|---|---|---|---|---|---|
| | | (g/m²) | (g/m²) | Skin side layer | Non-skin side layer | (mm) | (sec) | (g) |
| Example 1 | Up | 232 | 232 | 100 | 0 | 7.0 | 56 | 53 |
| Reference Example 1 | Down | 232 | 232 | 100 | 0 | 7.3 | 72 | 54 |

### [Table 2]

**Table 2**

| No. | Direction of groove portion | Basis weight of pulp fibers | Basis weight of SAP | SAP composition ratio (%) | | Thickness change (160 ml) | Absorption rate (240 ml) | Liquid return amount (240 ml) |
|---|---|---|---|---|---|---|---|---|
| | | (g/m²) | (g/m²) | Skin side layer | Non-skin side layer | (mm) | (sec) | (g) |
| Example 2 | Up | 278 | 182 | 100 | 0 | 6.2 | 76 | 64 |
| Example 3 | Up | 278 | 182 | 90 | 10 | 6.1 | 67 | 67 |
| Example 4 | Up | 278 | 182 | 80 | 20 | 6.2 | 75 | 73 |
| Example 5 | Up | 278 | 182 | 70 | 30 | 6.7 | 84 | 73 |
| Example 6 | Up | 278 | 182 | 60 | 40 | 6.6 | 95 | 76 |
| Comparative Example 1 | Up | 278 | 182 | 50 | 50 | 6.9 | 96 | 77 |

In Example 1 and Reference Example 1, the directions of the groove portions are different from each other. That is, in Example 1, the groove portions and the base portions are formed in the skin side layer of the absorbent core, and the groove portion corresponding portions and the base corresponding portions are formed in the non-skin side layer. In contrast, in Reference Example 1, the groove portions and the base portions are formed in the non-skin side layer of the absorbent core, and the groove portion corresponding portions and the base corresponding portions are formed in the skin side layer. When the SAP composition ratios of both the skin side layer and the non-skin side layer are combined, in Example 1, the average density of the SAP in the base portions is high, whereas in Reference Example 1, the average density of the SAP in the groove portion corresponding portions and the base corresponding portions is high. From the results of Table 1, it was confirmed that in all items of Example 1, the thickness change after absorption of 160 ml was small compared with Reference example 1, the absorption rate was excellent, and the result of the liquid return amount was equal to or less than the liquid return of Reference example 1. In Example 1, the groove portion corresponding portions and the base corresponding portions are arranged in a manner of continuing in the non-skin side layer, and in addition to the base corresponding portions, the groove portion corresponding portions contribute to diffusion. Further, in Example 1, since the absorption rate is faster than the absorption rate of Reference Example 1, it can be said that the diffusibility of the absorbent core is excellent. That is, in Example 1, the bodily fluid can be diffused by transporting the bodily fluid in the lengthwise direction of the absorbent core by the groove portions formed in the skin side layer. Since the non-skin side layer has the groove portion corresponding portion in addition to the base corresponding portion, and the base corresponding portion and the groove portion corresponding portion do not contain SAP, the bodily fluid can be more reliably diffused. Further, it was found that by diffusing the bodily fluid and then absorbing the bodily fluid in the base portions, an increase in the overall thickness can be suppressed even when the bodily fluid is repeatedly absorbed, the absorption rate among the absorption performance can be enhanced, and the rewettability can be maintained.

On the other hand, in a case of Reference Example 1, since the skin side layer contains a large amount of SAP, the bodily fluid is less likely to pass to the groove portions through the groove portion corresponding portions of the skin side layer. Further, in Reference Example 1, there is no SAP contained in the non-skin side layer, and the base potion, substantially formed of only pulp fibers, contracts in volume due to water absorption, and also the groove portion contracts. Therefore, in Reference Example 1, the thickness change was relatively small by an amount corresponding to the delay in the passage of water to the groove portions, but the thickness change was still larger than the thickness change in Example 1. As for Reference Example 1, a configuration in which the amount of SAP of the groove portion corresponding portion is reduced and the amounts of SAP of the base potion and the base corresponding portion are increased correspondingly can be considered as a comparative example with respect to Example 1. In such a case, in the comparative example, the density of SAP in the base corresponding portion is highest, and then the average density of SAP in the groove portion corresponding portion and the base potion is similarly low. In the comparative example, the base corresponding portion absorbs a larger amount of bodily fluid than in Reference Example 1, the densities of SAP of the base corresponding portion and the base potion are higher than in Reference Example 1, and the volume of the groove portion is increased due to water absorption. Therefore, the thickness becomes larger than in Reference Example 1. Therefore, in the configuration having the groove portions in the non-skin side layer, it can be said that the thickness is likely to become larger than the thickness in Example 1.

Examples 2 to 6 are examples in which the first average density of the non-skin side layer is lower than the second average density of the skin side layer. Comparative Example 1 is an example in which the first average density of the non-skin side layer and the second average density of the skin side layer are the same. In Examples 2 to 6, it was confirmed that the thickness change was small compared with Comparative Example 1, and particularly, in Examples 2 to 4 in which the SAP composition ratio in the non-skin side layer was 20% or less, the thickness change was small. That is, it was found that by reducing the amount of the superabsorbent polymer particles contained in the groove portion corresponding portions and the base corresponding portions compared to the base portions, the swelling of the groove portion corresponding portions and the base corresponding portions could be suppressed, and the absorption performance (absorption rate, rewettability) could be maintained.

### REFERENCE SIGNS LIST

1: absorbent article
6A, 6B: skin side layer
8: non-skin side layer
9A, 9B: absorbent core
18: Groove portion
19: Main groove portion
20A, 20B: base potion
21: sub-groove portion
22: main groove portion corresponding portion (groove portion corresponding portion)
24: base corresponding portion
26: sub-groove portion corresponding portion (groove portion corresponding portion)
31: narrow portion
32: wide portion
40: surface layer region
42: central region

## Claims

1. An absorbent article (1) having a lengthwise direction (L), a lateral direction (W), and a thickness direction (T), the absorbent article (1) comprising:
an absorbent core (9A) that contains superabsorbent polymer particles, wherein
the absorbent core (9A) has a skin facing surface (15), and a non-skin facing surface (17), and is formed of a skin side layer (6A) on a skin facing surface side, and a non-skin side layer (8) on a non-skin facing surface side,
the skin side layer (6A) includes
a plurality of groove portions (18) that extend in the lengthwise direction (L) and penetrate in the thickness direction (T), and
a plurality of base portions (20A) that extend in the lengthwise direction (L),
each of the plurality of groove portions (18) and each of the plurality of base portions (20A) alternately extend in the lateral direction (W),
the plurality of groove portions (18) include
a plurality of main groove portions (19) that extend in the lengthwise direction (L), and
a plurality of sub-groove portions (21) that are present in communication with each of the plurality of main groove portions (19) through base ends with predetermined spaces in a direction crossing each of the plurality of main groove portions (19),
the non-skin side layer (8) includes a plurality of groove portion corresponding portions (36) and a plurality of base corresponding portions (24) at positions that overlap the plurality of groove portions (18) and the plurality of base portions (20A) in the thickness direction (T), respectively, and
a first average density of the superabsorbent polymer particles contained in each of the plurality of groove portion corresponding portions (36) and the plurality of base corresponding portions (24) is lower than a second average density of the superabsorbent polymer particles contained in each of the plurality of base portions (20A).

2. The absorbent article (1) according to Claim 1, wherein
a ratio of the superabsorbent polymer particles contained in the plurality of groove portion corresponding portions (36) and the plurality of base corresponding portions (24) to the superabsorbent polymer particles contained in the entire absorbent core (9A) is less than 30%.

3. The absorbent article (1) according to Claim 1 or 2, wherein
each of the plurality of base portions (20A) has a surface layer region (40) including base side surfaces (35) that are in contact with the plurality of groove portions (18), and a top surface (34) that is positioned between the base side surfaces (35), and
an average density of the superabsorbent polymer particles contained in the surface layer region (40) is lower than the second average density.

4. The absorbent article (1) according to any one of Claims 1 to 3, wherein
in an excretion region of the absorbent core (9A), a space (L1) between the plurality of main groove portions (19) arranged on both sides in the lateral direction (W) with a center of the absorbent core (9A) in the lateral direction (W) interposed in between is 15% or more and less than 45% of a minimum length (L2) of the absorbent core (9A) in the lateral direction (W) in the excretion region.

5. The absorbent article (1) according to any one of Claims 1 to 4, wherein
the plurality of base portions (20A), the plurality of groove portion corresponding portions (36), and the plurality of base corresponding portions (24) are integrated.

6. The absorbent article (1) according to any one of Claims 1 to 5, wherein
an average density of the superabsorbent polymer particles contained in a central region (42) including a center of each of the plurality of base portions (20A) in the lateral direction (W) is higher than the second average density.

7. The absorbent article according (1) to any one of Claims 1 to 6, wherein
the plurality of base portions (20A) include
a plurality of narrow portions (31) in which the plurality of sub-groove portions (21) that are present in communication with each of the plurality of main groove portions (19) adjacent to each other in the lateral direction (W) face each other and which are present between the plurality of sub-groove portions (21) facing each other, and
a plurality of wide portions (32) which are present in a section of the plurality of base portions (20A) excluding the plurality of narrow portions (31).

8. The absorbent article (1) according to any one of Claims 1 to 7, wherein
the plurality of groove portion corresponding portions (36) include
a plurality of main groove portion corresponding portions (22) corresponding to the plurality of main groove portions (19), and
a plurality of sub-groove portion corresponding portions (26) corresponding to the plurality of sub-groove portions (21), and
the first average density of the superabsorbent polymer particles in the plurality of main groove portion corresponding portions (22) is lower than the second average density.

9. The absorbent article (1) according to Claim 8, wherein
the first average density of the plurality of sub-groove portion corresponding portions (26) is lower than the second average density.

10. The absorbent article (1) according to Claim 6, wherein
each of the plurality of base portions (20A) is continuous from one end (43) to the other end (44) of the absorbent core (9A) in the lengthwise direction.

## Patentansprüche

1. Absorbierender Artikel (1) mit einer Längsrichtung (L), einer Seitenrichtung (W) und einer Dickenrichtung (T), wobei der absorbierende Artikel (1) umfasst:
einen absorbierenden Kern (9A), der superabsorbierende Polymerpartikel enthält, wobei
der absorbierende Kern (9A) eine hautzugewandte Oberfläche (15) und eine hautabgewandte Oberfläche (17) aufweist und aus einer hautseitigen Schicht (6A) auf einer hautzugewandten Oberflächenseite und einer nichthautseitigen Schicht (8) auf einer hautabgewandten Oberflächenseite gebildet ist,
wobei die hautseitige Schicht (6A) einschließt:
eine Mehrzahl von Nutabschnitten (18), die sich in Längsrichtung (L) erstrecken und in der Dickenrichtung (T) durchdringen, und
eine Mehrzahl von Basisabschnitten (20A), die sich in Längsrichtung (L) erstrecken,
wobei jeder der Mehrzahl von Nutabschnitten (18) und jeder der Mehrzahl von Basisabschnitten (20A) sich abwechselnd in Seitenrichtung (W) erstrecken,
wobei die Mehrzahl von Nutabschnitten (18) einschließt:
eine Mehrzahl von Hauptnutabschnitten (19), die sich in Längsrichtung (L) erstrecken, und
eine Mehrzahl von Teilnutabschnitten (21), die mit jedem der Mehrzahl von Hauptnutabschnitten (19) durch Basisenden mit vordefinierten Abständen in einer Richtung in Kommunikation stehen, die jeden der Mehrzahl von Hauptnutabschnitten (19) kreuzt,
die nichthautseitige Schicht (8) eine Mehrzahl von Nutabschnitt, die Abschnitten (36) entsprechen, und eine Mehrzahl von basisentsprechenden Abschnitten (24) an Positionen einschließt, die die Mehrzahl von Nutabschnitten (18) bzw. die Mehrzahl von Basisabschnitten (20A) in Dickenrichtung (T) überlappen, und
eine erste durchschnittliche Dichte der superabsorbierenden Polymerpartikel, die in jedem der Mehrzahl von nutabschnittentsprechenden Abschnitten (36) und der Mehrzahl von basisentsprechenden Abschnitten (24) enthalten sind, geringer als eine zweite durchschnittliche Dichte der superabsorbierenden Polymerpartikel ist, die in jedem der Mehrzahl von Basisabschnitten (20A) enthalten sind.

2. Absorbierender Artikel (1) nach Anspruch 1, wobei
ein Verhältnis der superabsorbierenden Polymerpartikel, die in der Mehrzahl von nutabschnittentsprechenden Abschnitten (36) und der Mehrzahl von basisentsprechenden Abschnitten (24) enthalten sind, zu den superabsorbierenden Polymerpartikeln, die in dem gesamten absorbierenden Kern (9A) enthalten sind, weniger als 30 % beträgt.

3. Absorbierender Artikel (1) nach Anspruch 1 oder 2, wobei
jeder der Mehrzahl von Basisabschnitten (20A) eine Oberflächenschichtregion (40) mit basisseitigen Oberflächen (35), die mit der Mehrzahl von Nutabschnitten (18) in Kontakt stehen, und eine obere Oberfläche (24) aufweist, die zwischen den basisseitigen Oberflächen (35) positioniert ist, und
eine durchschnittliche Dichte der superabsorbierenden Polymerpartikel, die in der Oberflächenschichtregion (40) enthalten sind, geringer als eine zweite durchschnittliche Dichte ist.

4. Absorbierender Artikel (1) nach einem der Ansprüche 1 bis 3, wobei
in einer Ausscheideregion des absorbierenden Kerns (9A) ein Abstand (L1) zwischen der Mehrzahl von Hauptnutabschnitten (19) an beiden Seiten in Seitenrichtung (W), wobei eine Mitte des absorbierenden Kerns (9A) in der Seitenrichtung (W) dazwischen angeordnet ist, 15 % oder mehr und weniger als 45 % einer Mindestlänge (L2) des absorbierenden Kerns (9A) in Seitenrichtung (W) in der Ausscheideregion beträgt.

5. Absorbierender Artikel (1) nach einem der Ansprüche 1 bis 4, wobei
die Mehrzahl von Basisabschnitten (20A), die Mehrzahl von nutabschnittentsprechenden Abschnitten (36) und die Mehrzahl von basisentsprechenden Abschnitten (24) eingebunden sind.

6. Absorbierender Artikel (1) nach einem der Ansprüche 1 bis 5, wobei
eine durchschnittliche Dichte der superabsorbierenden Polymerpartikel, die in einer mittleren Region (42) einschließlich einer Mitte von jedem der Mehrzahl von Basisabschnitten (20A) in Seitenrichtung (W) enthalten sind, höher als die zweite durchschnittliche Dichte ist.

7. Absorbierender Artikel nach (1) einem der Ansprüche 1 bis 6, wobei
die Mehrzahl von Basisabschnitten (20A) einschließt
eine Mehrzahl von schmalen Abschnitten (31), in denen die Mehrzahl von Teilnutabschnitten (21), die mit jedem der Mehrzahl von Hauptnutabschnitten (19) einander in Seitenrichtung (W) benachbart in Kommunikation vorgesehen sind, einander zugewandt sind und zwischen der Mehrzahl von Teilnutabschnitten (21) vorgesehen sind, die einander zugewandt sind, und
eine Mehrzahl von breiten Abschnitten (32) die in einem Teilbereich der Mehrzahl von Basisabschnitten (20A) vorgesehen sind, ausschließlich der Mehrzahl von schmalen Abschnitten (31).

8. Absorbierender Artikel (1) nach einem der Ansprüche 1 bis 7, wobei
die Mehrzahl von nutenabschnittentsprechenden Abschnitten (36) einschließt:
eine Mehrzahl von hauptnutabschnittentsprechenden Abschnitten (22), die der Mehrzahl von Hauptnutabschnitten (19) entsprechen, und
eine Mehrzahl von teilnutabschnittentsprechenden Abschnitten (26), die der Mehrzahl von Teilnutabschnitten (19) entsprechen, und
die erste durchschnittliche Dichte der superabsorbierenden Polymerpartikel in der Mehrzahl von hauptnutabschnittentsprechenden Abschnitten (22) geringer als die zweite durchschnittliche Dichte ist.

9. Absorbierender Artikel (1) nach Anspruch 8, wobei
die erste durchschnittliche Dichte der Mehrzahl von teilnutabschnittentsprechenden Abschnitten (26) geringer als die zweite durchschnittliche Dichte ist.

10. Absorbierender Artikel (1) nach Anspruch 6, wobei
jeder der Mehrzahl von Basisabschnitten (20A) von einem Ende (43) zum anderen Ende (44) des absorbierenden Kerns (9A) in Längsrichtung durchgehend ist.

## Revendications

1. Article absorbant (1) ayant une direction longitudinale (L), une direction latérale (W) et une direction d'épaisseur (T), l'article absorbant (1) comprenant :
un noyau absorbant (9A) qui contient des particules polymères superabsorbantes, dans lequel
le noyau absorbant (9A) présente une surface tournée vers la peau (15) et une surface non tournée vers la peau (17), et est constitué d'une couche côté peau (6A) sur un côté de surface tournée vers la peau, et d'une couche non côté peau (8) sur un côté de surface non tournée vers la peau,
la couche côté peau (6A) comprend
une pluralité de parties de sillons (18) qui s'étendent dans la direction longitudinale (L) et pénètrent dans la direction d'épaisseur (T), et
une pluralité de parties de base (20A) qui s'étendent dans la direction longitudinale (L),
chacune de la pluralité de parties de sillons (18) et chacune de la pluralité de parties de base (20A) s'étend en alternance dans la direction latérale (W),
la pluralité de parties de sillons (18) comprend
une pluralité de parties de sillons principaux (19) qui s'étendent dans la direction longitudinale (L), et
une pluralité de parties de sous-sillons (21) qui sont présentes en communication avec chacune de la pluralité de parties de sillons principaux (19) à travers des extrémités de base avec des espaces prédéterminés dans une direction croisant chacune de la pluralité de parties de sillons principaux (19),
la couche non côté peau (8) comprend une pluralité de parties (36) correspondant aux parties de sillons et une pluralité de parties (24) correspondantes aux parties de base dans des positions qui se chevauchent avec la pluralité de parties de sillons (18) et la pluralité de parties de base (20A) dans la direction d'épaisseur (T), respectivement, et
une première densité moyenne des particules polymères superabsorbantes contenues dans chacune de la pluralité de parties (36) correspondant aux parties de sillons et de la pluralité de parties (24) correspondant aux parties de base est inférieure à une deuxième densité moyenne des particules polymères superabsorbantes contenues dans chacune de la pluralité de parties de base (20A).

2. Article absorbant (1) selon la revendication 1, dans lequel
un rapport entre les particules polymères superabsorbantes contenues dans la pluralité de parties (36) correspondant aux parties de sillons et la pluralité de parties (24) correspondant à la base et les particules polymères superabsorbantes contenues dans tout le noyau absorbant (9A) est inférieur à 30 %.

3. Article absorbant (1) selon la revendication 1 ou 2, dans lequel
chacune de la pluralité de parties de base (20A) présente une région de couche de surface (40) comprenant des surfaces côté base (35) qui sont en contact avec la pluralité de parties de sillons (18), et une surface supérieure (34) qui est positionnée entre les surfaces côté base (35), et
une densité moyenne des particules polymères superabsorbantes contenues dans la région de couche de surface (40) est inférieure à la deuxième densité moyenne.

4. Article absorbant (1) selon l'une quelconque des revendications 1 à 3, dans lequel
dans une région d'excrétion du noyau absorbant (9A), un espace (L1) entre la pluralité de parties de sillons principaux (19) agencées des deux côtés dans la direction latérale (W), un centre du noyau absorbant (9A) dans la direction latérale (W) étant interposé entre ceux-ci, mesure 15 % ou plus et moins de 45 % qu'une longueur minimale (L2) du noyau absorbant (9A) dans la direction latérale (W) dans la région d'excrétion.

5. Article absorbant (1) selon l'une quelconque des revendications 1 à 4, dans lequel
la pluralité de parties de base (20A), la pluralité de parties (36) correspondant aux parties de sillons et la pluralité de parties (24) correspondant à la base sont intégrées.

6. Article absorbant (1) selon l'une quelconque des revendications 1 à 5, dans lequel
une densité moyenne des particules polymères superabsorbantes contenues ans une région centrale (42) comprenant un centre de chacune de la pluralité de parties de base (20A) dans la direction latérale (W) est supérieure à la deuxième densité moyenne.

7. Article absorbant (1) selon l'une quelconque des revendications 1 à 6, dans lequel
la pluralité de parties de base (20A) comprend
une pluralité de parties étroites (31) dans lesquelles la pluralité de parties de sous-sillons (21) qui sont présentes en communication avec chacune de la pluralité de parties de sillons principaux (19) adjacentes les unes aux autres dans la direction latérale (W) se font face et qui sont présentes entre la pluralité de parties de sous-sillons (21) qui se font face, et
une pluralité de parties larges (32) qui sont présentes dans une section de la pluralité de parties de base (20A) à l'exclusion de la pluralité de parties étroites (31).

8. Article absorbant (1) selon l'une quelconque des revendications 1 à 7, dans lequel
la pluralité de parties (36) correspondant aux parties de sillons comprend
une pluralité de parties (22) correspondant aux parties de sillons principaux qui correspond à la pluralité de parties de sillons principaux (19), et
une pluralité de parties (26) correspondant aux parties de sous-sillons qui correspond à la pluralité de parties de sous-sillons (21), et
la première densité moyenne des particules polymères superabsorbantes dans la pluralité de parties (22) correspondant aux parties de sillons principaux est inférieure à la deuxième densité moyenne.

9. Article absorbant (1) selon la revendication 8, dans lequel
la première densité moyenne de la pluralité de parties (26) correspondant aux parties de sous-sillons est inférieure à la deuxième densité moyenne.

10. Article absorbant (1) selon la revendication 6, dans lequel
chacune de la pluralité de parties de base (20A) est continue d'une extrémité (43) à l'autre extrémité (44) du noyau absorbant (9A) dans la direction longitudinale.
